# FASCICULE DE BREVET EUROPEEN

(11) **EP 3 656 439 B1**
(45) Date de publication et mention de la délivrance du brevet: **13.10.2021**
(21) Numéro de dépôt: 19209454.8
(22) Date de dépôt: 15.11.2019
(51) Int. Cl.: A61N 1/05, H01R 4/22, H01R 4/60, H01R 4/62, H01R 43/02, H01R 4/02, B23K 26/211, B23K 26/32, B23K 26/20, B23K 101/38

(54) **SONDE IMPLANTABLE**
IMPLANTIERBARE SONDE
IMPLANTABLE PROBE

(30) Priorité: 21.11.2018 FR 1871664
(43) Date de publication de la demande: 27.05.2020
(73) Titulaire: Sorin CRM SAS, 92140 Clamart (FR)
(72) Inventeur: JULIEN, Etienne, 92310 Sèvres (FR); SHAN, Nicolas, 92160 Antony (FR); RAULT, Maxime, 75015 Paris (FR)
(74) Mandataire: Grünecker Patent- und Rechtsanwälte PartG mbB

(56) Documents cités:
- EP-A1- 3 075 411
- US-A1- 2010 179 627
- US-A1- 2011 220 408
- US-A1- 2015 165 217

## Description

La présente invention se rapporte à une sonde implantable, ainsi qu'à une méthode, pour connecter électriquement au moins un fil conducteur de la sonde implantable à un connecteur, en particulier à un connecteur destiné à être connecté à un dispositif de stimulation cardiaque, de défibrillation ou/et de neuromodulation.

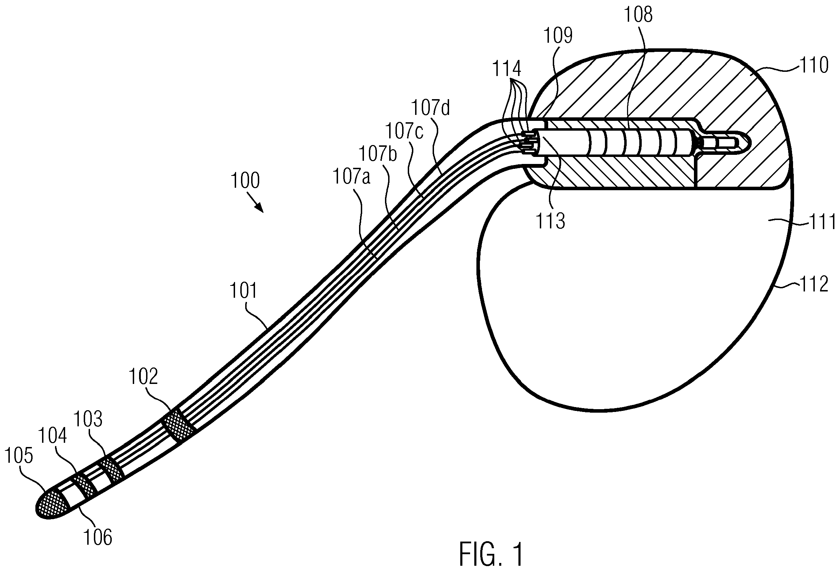

De telles sondes pour dispositifs médicaux implantables, telle que la sonde implantable 100 illustrée par la Figure 1, comprennent usuellement un corps de sonde 101 allongé avec quatre électrodes 102, 103, 104, 105 vers l'extrémité distale 106 configurées pour mesurer un ou plusieurs paramètres cardiaques et/ou stimuler le tissu cardiaque, ainsi que des fils conducteurs 107a-d et un connecteur 108 à l'extrémité proximale 109 pour permettre la connexion électrique avec un bloc de connexion 110 d'un boîtier 111 d'un dispositif médical implantable 112. La connexion électrique entre les fils conducteurs 107a-d de la sonde 100 et le connecteur 108 au niveau de l'extrémité proximale 113 du connecteur 108 est réalisée par l'intermédiaire d'un ou plusieurs hypotubes 114 eux-mêmes reliés électriquement à quatre contacts électriques (non visible sur la Figure 1) du connecteur 109. Les hypotubes 114 sont en général des tubes en alliage à usage médical de type MP35N, platine ou d'aciers inoxydables, couramment utilisés, par exemple dans la fabrication de dispositifs médicaux. La connexion électrique des fils conducteurs 107a-d au connecteur électrique 108 est communément réalisée par soudure, ou sertissage, des fils conducteurs 107a-d aux hypotubes 114 du connecteur 108.

Cependant, la soudure ou le sertissage des fils conducteurs médicaux sont des moyens de connexion susceptibles d'endommager les fils conducteurs de la sonde, notamment à cause du stress mécanique appliqué ou de l'énergie dégagée par ces moyens de connexion sur les fils conducteurs. Le risque d'endommagement des fils est notamment augmenté lorsque les fils conducteurs de la sonde ont un diamètre inférieur à 150 micromètres soit 0,45 French.

EP3075411 A1, US2011220408 A1, US2010179627 A1 et US2015165217 A1 montrent des sondes implantables connues comprenant au moins un fil conducteur et un connecteur électrique, ainsi que leur méthode de connexion. L'objet de la présente invention est ainsi de faciliter et de sécuriser la connexion électrique des fils conducteurs de la sonde au connecteur afin d'améliorer la fiabilité de la connexion électrique entre les fils électriques et le connecteur de la sonde.

L'objet de l'invention est atteint par une sonde implantable comprenant au moins un fil conducteur et un connecteur électrique, le connecteur électrique étant configuré pour être connecté à un dispositif médical implantable de type dispositif de stimulation cardiaque, de défibrillation ou/et de neuromodulation ; dans laquelle la connexion électrique entre le fil conducteur et le connecteur est réalisée au moyen d'un premier hypotube soudé au fil conducteur et soudé à un second hypotube du connecteur électrique. L'utilisation d'un premier hypotube, tel un intermédiaire, entre le fil conducteur et le second hypotube du connecteur électrique permet de simplifier et faciliter la réalisation de la connexion électrique entre le fil conducteur, qui devient de plus en plus fin, et le connecteur électrique.

La sonde implantable selon la présente invention peut être davantage améliorée grâce aux modes de réalisation suivants.

Selon une autre réalisation de l'invention, le premier hypotube peut être logé partiellement dans le second hypotube du connecteur électrique de manière à ce qu'une portion du premier hypotube fasse saillie hors du second hypotube. Les dimensions du premier hypotube permettent de s'accommoder de la différence de dimension entre le diamètre interne du second hypotube du connecteur et le diamètre du fil conducteur. De ce fait, le contact électrique peut facilement être réalisé entre les deux hypotubes lorsque le premier hypotube dépasse hors du second hypotube. De plus, le premier hypotube permet de faciliter la réalisation de la connexion électrique d'un fil conducteur à un connecteur, même lorsque le diamètre du fil conducteur est nettement inférieur au diamètre interne du second hypotube du connecteur, en particulier environ trois fois plus petit.

Selon une autre réalisation de l'invention, le premier hypotube peut être soudé au second hypotube du connecteur électrique de manière à connecter électriquement le premier hypotube au second hypotube. La connexion électrique entre le premier et le second hypotube peut ainsi être réalisée facilement, par le biais d'une soudure. De plus, lorsqu'une portion du premier hypotube fait saillie hors du second hypotube, la réalisation de la soudure entre le premier hypotube et le second hypotube est facilitée car elle peut être réalisée en dehors du second hypotube.

Selon une autre réalisation de l'invention, la soudure entre le au moins un fil conducteur et le premier hypotube peut être réalisée au niveau d'une première extrémité du premier hypotube par laquelle est insérée le au moins un fil conducteur et/ou au niveau d'une deuxième extrémité du premier hypotube, la deuxième extrémité étant opposée à la première extrémité. De ce fait, un opérateur a le choix quant à l'extrémité du premier hypotube sur laquelle (ou lesquelles) réaliser la soudure car la nature du contact électrique entre le premier hypotube et le fil conducteur s'avère être le même qu'importe l'extrémité du premier hypotube soudée au fil conducteur. De plus, lorsque la soudure est réalisée au niveau des deux extrémités du premier hypotube, le risque de dysfonctionnement, notamment en terme de connexion électrique, est réduit par la redondance de la soudure.

Selon une autre réalisation de l'invention, la sonde peut comprendre plusieurs fils conducteurs tel que chaque fil conducteur soit électriquement connecté à un premier hypotube respectif. Selon une variante, la sonde peut comprendre plusieurs fils conducteurs, tels qu'au moins deux fils conducteurs soient électriquement connectés à un même premier hypotube. De ce fait, le premier hypotube est aussi bien adapté pour être soudé à un fil conducteur qu'à au moins deux fils conducteurs, ce qui élargit les possibilités d'application du premier hypotube selon l'invention pour une sonde implantable.

Selon une autre réalisation de l'invention, la ou les soudures réalisées pour établir les connexions électriques peuvent être des soudures laser. La soudure au laser permet notamment de réaliser une soudure étanche, et permet également une soudure précise particulièrement adaptée à l'échelle de connecteurs de sondes implantables de dispositifs médicaux. De plus, la soudure au laser étant réalisée au niveau d'une ou des extrémités du premier hypotube, et non directement sur le fil conducteur, le risque d'endommager ou de détruire le fil conducteur à cause de l'énergie dégagée par le faisceau laser est réduit.

Selon une autre réalisation de l'invention, le au moins un fil conducteur peut être un fil conducteur monobrin ou multibrins tel que le fil conducteur a un diamètre inférieur à 150 micromètres. Le premier hypotube permet ainsi de pouvoir connecter un fil conducteur dont le diamètre est nettement plus petit que celui d'un hypotube du connecteur, en particulier trois à six fois plus petit pour un fil conducteur, et dont le diamètre interne standard d'un hypotube de connecteur est généralement compris entre 300 et 500 micromètres, ou entre 0,9 et 1,5 French. Ainsi, lors de la connexion électrique de fil conducteur au diamètre inférieur à 150 micromètres, le premier hypotube permet de s'accommoder de la différence de dimension entre le diamètre interne de l'hypotube du connecteur et le diamètre du fil conducteur.

L'objet de la présente invention est également atteint par une méthode pour connecter électriquement au moins un fil conducteur d'une sonde implantable à un connecteur électrique, le connecteur électrique étant configuré pour être connecté à un dispositif médical implantable de type dispositif de stimulation cardiaque, de défibrillation ou/et de neuromodulation, comprenant les étapes de : a) loger le au moins un fil conducteur de la sonde dans un premier hypotube; b) connecter électriquement le au moins un fil conducteur au premier hypotube ; c) loger au moins partiellement le premier hypotube dans un second hypotube correspondant du connecteur électrique ; d) connecter électriquement le premier hypotube avec le second hypotube. De ce fait, les premiers hypotubes permettent de pouvoir réaliser des connections électriques avec un risque réduit d'endommager directement les fils conducteurs de la sonde lors de la soudure. En effet, chaque fil conducteur de la sonde est préalablement logé dans un premier hypotube est ainsi protégé lors de la connexion électrique. De plus, cette étape de connexion électrique entre le fil conducteur et le premier hypotube est plus facile à réaliser qu'une connexion électrique lorsque le fil conducteur est directement introduit dans le second hypotube du connecteur. Ainsi, les premiers hypotubes permettent aussi de faciliter l'assemblage et la connexion électrique du fil conducteur au connecteur.

La présente invention, relative à une méthode pour connecter électriquement au moins un fil conducteur d'une sonde implantable à un connecteur électrique, peut être davantage améliorée grâce aux modes de réalisation suivants.

Selon une autre réalisation de l'invention, l'étape b) et/ou d) de la méthode peut comprendre la réalisation d'une soudure au laser. Ainsi, comme le fil conducteur est protégé par le premier hypotube, le faisceau laser de la soudure n'atteint pas directement le fil conducteur lui-même, ce qui pourrait - selon le diamètre du fil - l'endommager, ou le détruire. De plus, la soudure laser permet la formation d'une surface lisse et arrondie au niveau d'une ou des extrémités du premier hypotube, ce qui permet d'améliorer davantage la qualité de la connexion électrique.

Selon une autre réalisation de l'invention, la soudure à l'étape b) peut être réalisée au niveau d'une première extrémité du premier hypotube par laquelle est insérée le au moins un fil conducteur et/ou au niveau d'une deuxième extrémité du premier hypotube, la deuxième extrémité étant opposée à la première extrémité. De ce fait, un opérateur a le choix quant à l'extrémité du premier hypotube sur laquelle (ou lesquelles) réaliser la soudure car la nature du contact électrique entre le premier hypotube et le fil conducteur s'avère être le même qu'importe l'extrémité du premier hypotube soudée au fil conducteur. De plus, lorsque la soudure est réalisée au niveau des deux extrémités du premier hypotube, le risque de dysfonctionnement, notamment en terme de connexion électrique, est réduit par la redondance de la soudure.

Selon une autre réalisation de l'invention, l'étape c) de la méthode peut comprendre l'insertion du premier hypotube dans le second hypotube de manière à ce qu'une portion du premier hypotube fasse saillie hors du second hypotube. Ainsi, la réalisation de la soudure entre le premier hypotube et le second hypotube est facilitée car elle peut être réalisée en dehors du second hypotube, offrant davantage d'espace et de visibilité à un opérateur pour effectuer la soudure.

Les modes de réalisation peuvent être combinés pour former davantage de variantes de mode de réalisation avantageux de la présente invention.

L'invention et ses avantages seront expliqués plus en détail dans la suite au moyen de modes de réalisation préférés et en s'appuyant notamment sur les figures d'accompagnement suivantes, dans lesquelles :
Figure 1 représente schématiquement une sonde implantable selon l'art antérieur.
Figure 2a représente une vue partielle de la sonde implantable selon la présente invention.
Figure 2b représente une vue en coupe de la sonde implantable représentée à la Figure 2a.
Figure 3a représente une étape de la méthode pour connecter électriquement un fil conducteur d'une sonde implantable à un connecteur électrique selon un mode de réalisation de la présente invention.
Figure 3b représente une étape de la méthode pour connecter électriquement un fil conducteur d'une sonde implantable à un connecteur électrique selon un mode de réalisation de la présente invention.
Figure 3c représente une étape de la méthode pour connecter électriquement un fil conducteur d'une sonde implantable à un connecteur électrique selon un mode de réalisation de la présente invention.
Figure 3d représente une étape de la méthode pour connecter électriquement un fil conducteur d'une sonde implantable à un connecteur électrique selon un mode de réalisation de la présente invention.

L'invention va maintenant être décrite plus en détail en utilisant des modes de réalisation avantageux d'une manière exemplaire et en référence aux dessins. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

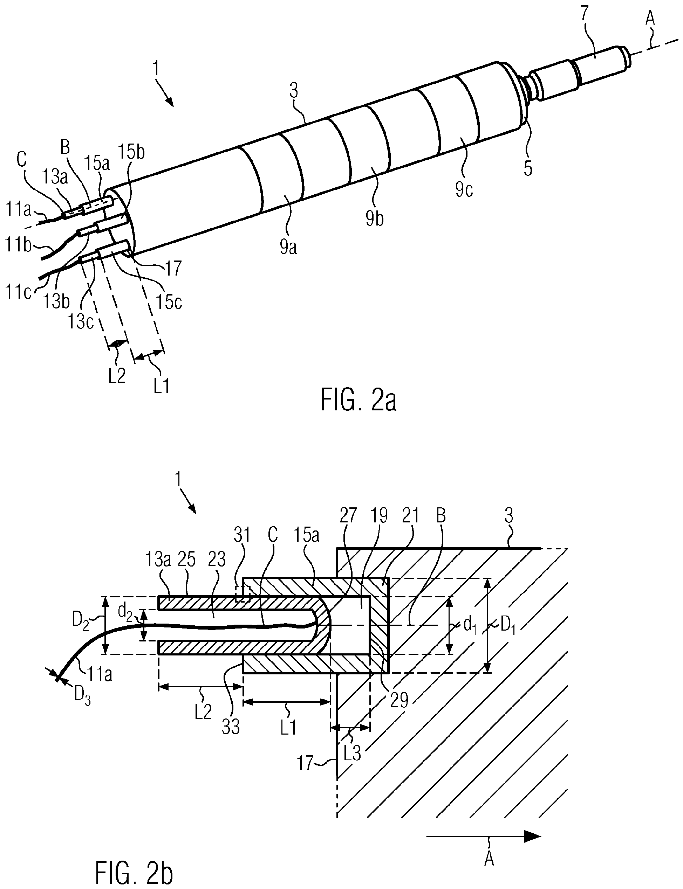

La Figure 2a illustre schématiquement une vue partielle d'une sonde implantable 1 au niveau de son extrémité proximale. La Figure 2a représente un connecteur 3 de la sonde implantable 1. Le connecteur 3 a une forme cylindrique d'axe A et comprend au niveau de son extrémité distale 5 une broche distale 7 configurée pour être connectée à un terminal d'un dispositif médical implantable (non représenté) tel un dispositif de stimulation cardiaque, de défibrillation ou/et de neuromodulation.

Le connecteur 3 représenté à la Figure 2a est de type multipolaire car il est pourvu de trois contacts 9a, 9b, 9c. Dans une variante, le connecteur 3 peut comprendre plus ou moins que trois contacts. Dans une autre variante, le connecteur 3 peut comprendre un seul contact.

Le connecteur 3 peut être réalisé à partir de matériaux conducteurs biocompatibles tels que l'acier inoxydable 316L ou un alliage métallique, MP35N, par exemple. Le matériau du connecteur 3 peut être sélectionné pour être biocompatible et conduire et transmettre de manière appropriée des signaux électriques provenant d'un dispositif de stimulation électrique (non représenté).

La sonde implantable 1 comprend également des fils conducteurs 11a, 11b, 11c qui permettent de connecter électriquement les contacts 9a, 9b, 9c du connecteur 3 à une ou des électrodes (non représentés) de la sonde 1. Dans le mode de réalisation illustré par la Figure 2a, les fils conducteurs 11a, 11b, 11c sont chacun des fils conducteurs multibrins tel que le diamètre de chaque fil conducteur multibrin 11a, 11a, 11c est inférieur à 150 micromètres. Les fils conducteurs individuels constituant chacun des fils conducteurs multibrins 11a, 11b, 11c ne sont pas électriquement isolés des uns des autres. Toutefois, les fils conducteurs multibrins 11a, 11b, 11c sont, quant à eux, isolés électriquement des uns des autres.

Dans une variante, les fils conducteurs 11a, 11b, 11c sont des fils conducteurs monobrins tel que le diamètre de chaque fil conducteur 11a, 11b, 11c, c'est-à-dire de chaque fil conducteur monobrin, est inférieur à 150 micromètres. De plus, les fils conducteurs monobrins 11a, 11b, 11c sont isolés électriquement des uns des autres.

Selon la présente invention, les fils conducteurs 11a, 11b, 11c sont chacun logés et soudés à un premier hypotube 13a, 13b, 13c ; et chaque premier hypotube 13a, 13b, 13c est lui-même logé et soudé à un second hypotube 15a, 15b, 15c du connecteur 3 au niveau de l'extrémité proximale 17 du connecteur 3.

L'axe A du connecteur 3 est parallèle aux axes B, C respectifs des seconds hypotubes 15a, 15b, 15c et des premiers hypotube 13a, 13b, 13c. L'axe C de chaque premier hypotube 13a, 13b, 13c est confondu avec l'axe B du second hypotube 15a, 15b, 15c dans lequel il est inséré.

Les seconds hypotubes 15a, 15b, 15c sont logés dans le connecteur 3 et reliés électriquement avec les contacts 9a, 9b, 9c. Une portion de longueur L1 de chaque second hypotube 15a, 15b, 15c fait saillie hors du connecteur 3.

Les premiers hypotubes 13a, 13b, 13c sont logés dans un second hypotube correspondant 15a, 15b, 15c de manière à ce qu'une portion de longueur L2 de chaque premier hypotube 13a, 13b, 13c fasse saillie hors du second hypotube 15a, 15b, 15c.

Dans une variante, plusieurs fils conducteurs peuvent être logés dans un même premier hypotube. Le nombre de premiers hypotubes, de second hypotubes et de fils conducteurs, qui sont chacun au nombre de trois dans la sonde implantable 1 représentée en Figure 2a, est ainsi illustratif et ne constitue pas une limitation numérique de la présente invention.

La Figure 2a sera davantage décrite dans ce qui suit en combinaison avec la Figure 2b qui illustre une vue partielle en coupe de la sonde 1. Quand bien même la Figure 2b illustre un seul des premier et second hypotubes 13a, 15a de la Figure 2a, la description des hypotubes 13a, 15a s'applique également aux autres hypotubes 13b, 13c, 15b, 15c de la sonde implantable 1. Les éléments avec les mêmes références numériques déjà utilisées pour la description de la Figure 2a ne seront pas décrits à nouveau en détail, et référence est faite à leurs descriptions ci-dessus.

La Figure 2b représente le second hypotube 15a qui est partiellement logé dans le connecteur 3. Le second hypotube 15a est connecté électriquement (non représenté) à un des contacts 9a, 9b, 9c du connecteur 3. Le second hypotube 15a comprend une partie creuse 19 de diamètre interne d1 et une paroi 21 en acier inoxydable. Le second hypotube 15a a un diamètre externe D1.

Le premier hypotube 13a comprend lui aussi une partie creuse 23 qui est de diamètre interne d2 et une paroi 25 en acier inoxydable. Le second hypotube 15a a un diamètre externe D2.

Le diamètre externe D2 du premier hypotube 13a est inférieur ou égal au diamètre interne d1 du second hypotube 15a afin qu'il soit possible d'insérer le premier hypotube 13a dans la partie creuse 19 du second hypotube 15a. Tel qu'illustré à la Figure 2b, le diamètre externe D2 du premier hypotube 13a est dimensionné de manière à ce que la paroi 25 du premier hypotube 13a soit en contact avec la paroi 21 du second hypotube 15a afin d'améliorer le contact électrique et le maintien du premier hypotube 13a dans le second hypotube 15a.

Le diamètre D3 du fil conducteur 11a est inférieur au diamètre interne d2 du premier hypotube 13a. En particulier, le fil conducteur 11a selon la présente invention a un diamètre D3 inférieur à 150 micromètres. Le diamètre interne d2 du premier hypotube 13a est quant à lui compris entre 150 micromètres et 350 micromètres. Ainsi, les dimensions d2, D2 du premier hypotube 13a permettent de s'accommoder de la différence de dimension entre le diamètre interne d1 du second hypotube 15a du connecteur 3 et le diamètre D3 du fil conducteur 11a. De ce fait, le premier hypotube 13a, en plus de permettre une connexion électrique, est aussi un moyen d'adaptation pour s'accommoder de la différence de dimension entre le fil conducteur 11a et le second hypotube 15a de connecteur 3.

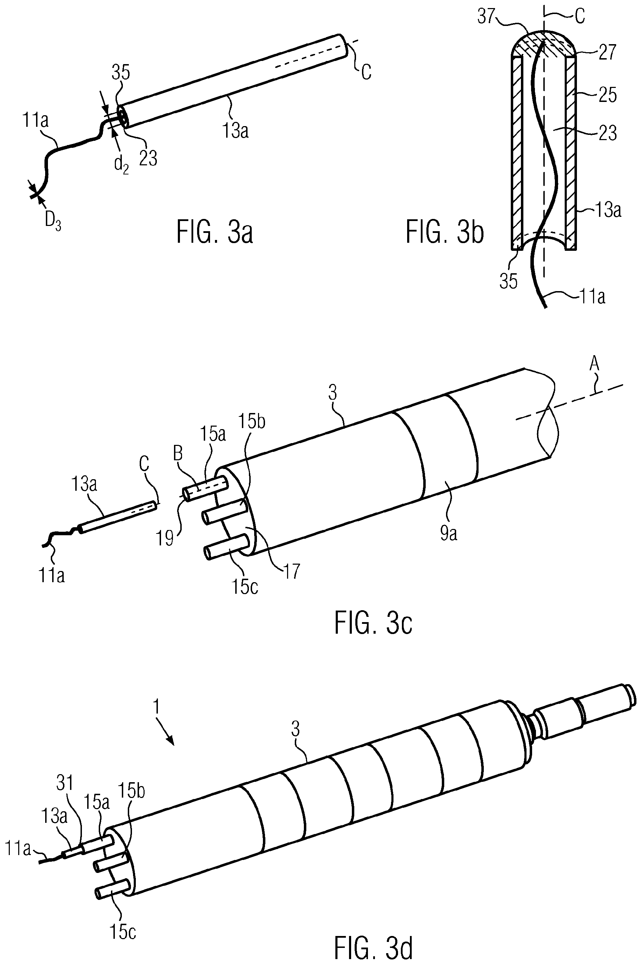

Dans le mode de réalisation illustré par la Figure 2b, le fil conducteur 11a est inséré dans la partie creuse 23 du premier hypotube 13a et est soudé au niveau d'une extrémité 27 du premier hypotube 13a. La soudure du fil conducteur 11a au premier hypotube 13a sera davantage décrite en référence à la Figure 3b.

Dans le mode de réalisation illustré par la Figure 2b, le premier hypotube 13a est partiellement logé dans le second hypotube 15a de sorte qu'il y ait une distance L3 entre l'extrémité 27 du premier hypotube 13a et une extrémité fermée 29 du second hypotube 15a.

Selon un autre mode de réalisation, le premier hypotube 13a peut être inséré jusqu'en butée dans le second hypotube 15a, de manière à ce qu'il y ait un contact entre l'extrémité 27 du premier hypotube 13a et l'extrémité fermée 29 du second hypotube 15a. Dans une variante, non-illustrée par la Figure 2b, l'extrémité 29 du second hypotube 15a peut être une extrémité partiellement fermée ou une extrémité ouverte.

Le premier hypotube 13a est soudé au second hypotube 15a en dehors du connecteur 3 au niveau d'une zone de jonction 31 située entre la paroi 25 du premier hypotube 13a et une extrémité ouverte 33 du second hypotube 15a. Ainsi, la réalisation de la soudure entre le premier hypotube 13a et le second hypotube 15a est facilitée car elle peut être réalisée en dehors du second hypotube 15a et du connecteur 3, offrant davantage d'espace et de visibilité à un opérateur pour effectuer la soudure. De plus, la zone de soudure 31 entre le premier hypotube 13a et le second hypotube 15a correspondant est distincte des zones de soudure des autre premiers hypotubes 13b, 13c et de leurs seconds hypotubes 15b, 15c respectifs (voir Figure 2a, non représenté sur la Figure 2b). Ainsi, un opérateur peut, par exemple, s'affairer à la connexion électrique au niveau du premier hypotube 13a sans pour autant avoir à retravailler les connexions électriques des autres premiers hypotubes 13b, 13c.

Selon une réalisation de la présente invention, la soudure entre le fil conducteur 11a et le premier hypotube 13a, ainsi que la soudure au niveau de la zone de jonction 31 entre le premier hypotube 13a et le second hypotube 15a sont réalisées par soudure laser. La soudure au laser permet notamment de réaliser une soudure étanche, et permet également une soudure précise particulièrement adaptée à l'échelle de connecteurs de sondes implantables de dispositifs médicaux.

Les Figures 3a à 3d illustrent schématiquement des étapes de la méthode pour connecter électriquement le fil conducteur 11a de la sonde implantable 1 au connecteur 3 selon un autre mode de réalisation.

Les éléments avec les mêmes références numériques déjà utilisées pour la description des Figures 2a et 2b ne seront pas décrits à nouveau en détail, et référence est faite à leurs descriptions ci-dessus.

La Figure 3a illustre la première étape à laquelle le fil conducteur 11a est inséré par une extrémité ouverte 35 du premier hypotube 13a dans la partie creuse 23 du premier hypotube 13a. Dans une variante, plusieurs fils conducteurs peuvent être introduits dans le premier hypotube 13a.

Selon le mode de réalisation illustré par la Figure 3a, le fil conducteur 11a à un diamètre D3 inférieur à 150 micromètres et la partie creuse 23 du premier hypotube 13a à un diamètre interne d2 compris entre 150 et 350 micromètres.

La Figure 3b illustre une vue en coupe longitudinale selon l'axe C du premier hypotube 13a à l'étape où le fil conducteur 11a est soudé au premier hypotube 13a afin de les connecter électriquement.

Selon le mode de réalisation illustré par la Figure 3b, le fil conducteur 11a a été introduit par l'extrémité ouverte 35 du premier hypotube 13a jusqu'à l'extrémité 27 opposée à l'extrémité 35 ; et soudé au niveau de l'extrémité 27 du premier hypotube 13a.

Selon d'autres modes de réalisation, le fil conducteur 11a peut être soudé au niveau de l'extrémité 35 du premier hypotube 13a, par laquelle le conducteur 11a est introduit, ou aux deux extrémités 27, 35 du premier hypotube 13a, tant qu'une connexion électrique entre le fil conducteur 11a et l'hypotube 13a est permise.

La soudure entre le fil conducteur 11a et le premier hypotube 13a est réalisée par une soudure laser. Ainsi, une surface lisse et arrondie 37 est formée à l'extrémité 27 du premier hypotube 13a et le contact électrique entre le fil conducteur 11a et le premier hypotube 13a est obtenu.

Le faisceau laser n'étant pas dirigé et appliqué directement sur le fil conducteur 11a, mais sur une des extrémités 27, 35 du premier hypotube 13a dans lequel le fil conducteur 11a est logé, le risque d'endommager ou de détruire le fil conducteur 11a, notamment lorsque son diamètre est inférieur à 150 micromètres, à cause de l'énergie dégagée par le faisceau laser, est réduit.

La Figure 3c illustre l'étape d'insertion du premier hypotube 13a auquel est soudé le fil conducteur 11a.

L'extrémité 27 du premier hypotube 13a est insérée dans la partie creuse 19 du second hypotube 19 de manière à ce qu'une portion de longueur L2 du premier hypotube 13a reste en dehors du second hypotube 15a, tel qu'illustré aux Figures 2a et 2b.

L'étape suivante de la méthode pour connecter électriquement le fil conducteur 11a de la sonde implantable 1 au connecteur 3 est illustrée par la Figure 3d.

Cette étape consiste à souder le premier hypotube 13a au second hypotube 15a au niveau de la jonction 31 située en dehors du connecteur 3. Cette étape permet de connecter électriquement le second hypotube 15a du connecteur 3 au premier hypotube 13a, lui-même électriquement connecté au fil conducteur 11a lors de l'étape illustrée par la Figure 3b. Ainsi, le premier hypotube 13a sert d'intermédiaire à la connexion électrique entre le fil conducteur 11a et le second hypotube 15a du connecteur 3. L'utilisation du premier hypotube 13a, permettant de faire l'intermédiaire entre le fil conducteur 11a et le connecteur 3, rend alors possible une connexion électrique entre un connecteur comprenant un hypotube aux dimensions standards comprises entre 350 et 500 micromètres et un fil conducteur de diamètre inférieur à 150 micromètres.

La description des étapes pour connecter électriquement le fil conducteur 11a au connecteur 3 de la sonde 1 en référence aux Figures 3a à 3d s'applique de manière intégrale à la connexion des fils conducteurs 11b et 11c au connecteur 3 de la sonde 1.

L'homme de l'art appréciera que la présente invention puisse être appliquée essentiellement à tout type de sonde implantable dont le connecteur électrique est pourvu d'au moins un hypotube.

## Revendications

1. Sonde implantable comprenant au moins un fil conducteur (11a, 11b, 11c) et un connecteur électrique (3), le connecteur électrique (3) étant configuré pour être connecté à un dispositif médical implantable de type dispositif de stimulation cardiaque, de défibrillation ou/et de neuromodulation ; dans laquelle la connexion électrique entre le fil conducteur (11a, 11b, 11c) et le connecteur (3) est réalisée au moyen d'un premier hypotube (13a, 13b, 13c) soudé au fil conducteur (11a, 11b, 11c) **caractérisée en ce que**:
le premier hypotube (13a, 13b, 13c) est soudé à un second hypotube (15a, 15b, 15c) du connecteur électrique (3).

2. La sonde implantable selon la revendication 1, dans laquelle le premier hypotube (13a, 13b, 13c) est logé partiellement dans le second hypotube (15a, 15b, 15c) du connecteur électrique (3) de manière à ce qu'une portion (L2) du premier hypotube (13a, 13b, 13c) fasse saillie hors du second hypotube (15a, 15b, 15c).

3. La sonde implantable selon la revendication 1 ou 2, dans laquelle le premier hypotube (13a, 13b, 13c) est soudé au second hypotube (15a, 15b, 15c) du connecteur électrique (3) de manière à connecter électriquement le premier hypotube (13a, 13b, 13c) au second hypotube (15a, 15b, 15c).

4. La sonde implantable selon la revendication 3, dans laquelle la soudure entre le au moins un fil conducteur (11a, 11b, 11c) et le premier hypotube (13a, 13b, 13c) est réalisée au niveau d'une première extrémité (27) du premier hypotube (13a, 13b, 13c) par laquelle est insérée le au moins un fil conducteur (11a, 11b, 11c) et/ou au niveau d'une deuxième extrémité (35) du premier hypotube (13a, 13b, 13c), la deuxième extrémité (35) étant opposée à la première extrémité (27).

5. La sonde implantable selon l'une des revendications 1 à 4, comprenant plusieurs fils conducteurs (11a, 11b, 11c), tel que chaque fil conducteur (11a, 11b, 11c) est électriquement connecté à un premier hypotube respectif (13a, 13b, 13c).

6. La sonde implantable selon l'une des revendications 1 à 4, comprenant plusieurs fils conducteurs (11a, 11b, 11c), tel qu'au moins deux fils conducteurs (11a, 11b, 11c) sont électriquement connectés à un même premier hypotube (13a, 13b, 13c).

7. La sonde implantable selon l'une quelconque des revendications précédentes, dont la ou les soudures réalisées pour établir les connexions électriques est/sont une/des soudure(s) laser.

8. La sonde implantable selon l'une quelconque des revendications précédentes, dont le au moins un fil conducteur est un fil conducteur monobrin ou multibrins (11a, 11b, 11c) tel que le fil conducteur a un diamètre inférieur à 150 micromètres ou 0,45 French.

9. Méthode pour connecter électriquement au moins un fil conducteur (11a, 11b, 11c) d'une sonde implantable (1) à un connecteur électrique (3), le connecteur électrique (3) étant configuré pour être connecté à un dispositif médical implantable de type dispositif de stimulation cardiaque, de défibrillation ou/et de neuromodulation, comprenant les étapes de :
a) Loger le au moins un fil conducteur (11a, 11b, 11c) de la sonde (1) dans un premier hypotube (13a, 13b, 13c);
b) Connecter électriquement le au moins un fil conducteur (11a, 11b, 11c) au premier hypotube (13a, 13b, 13c) ;
**caractérisée par** les étapes suivantes:
c) Loger au moins partiellement le premier hypotube (13a, 13b, 13c) dans un second hypotube (15a, 15b, 15c) correspondant du connecteur électrique (3) ;
d) Connecter électriquement le premier hypotube (13a, 13b, 13c) avec le second hypotube (15a, 15b, 15c).

10. La méthode pour connecter électriquement au moins un fil conducteur (11a, 11b, 11c) d'une sonde implantable (1) à un connecteur électrique (3) selon la revendication 9, dont l'étape b) et/ou d) comprend la réalisation d'une soudure au laser.

11. La méthode pour connecter électriquement au moins un fil conducteur (11a, 11b, 11c) d'une sonde implantable (1) à un connecteur électrique (3) selon la revendication 9 ou 10, dont la soudure à l'étape b) est réalisée au niveau d'une première extrémité (27) du premier hypotube (13a, 13b, 13c) par laquelle est insérée le au moins un fil conducteur (11a, 11b, 11c) et/ou au niveau d'une deuxième extrémité (35) du premier hypotube (13a, 13b, 13c), la deuxième extrémité (35) étant opposée à la première extrémité (27).

12. La méthode pour connecter électriquement au moins un fil conducteur (11a, 11b, 11c) d'une sonde implantable (1) à un connecteur électrique (3) selon l'une des revendications 9 à 11, dont l'étape c) comprend l'insertion du premier hypotube (13a, 13b, 13c) dans le second hypotube (15a, 15b, 15c) de manière à ce qu'une portion (L2) du premier hypotube (13a, 13b, 13c) fasse saillie hors du second hypotube (15a, 15b, 15c).

## Patentansprüche

1. Implantierbare Sonde, die mindestens einen Sondendraht (11a, 11b, 11c) und einen elektrischen Verbinder (3) umfasst, wobei der elektrische Verbinder (3) so konfiguriert ist, dass er mit einer implantierbaren medizinischen Vorrichtung wie einer Herzstimulations-, Defibrillations- oder/und Neuromodulationsvorrichtung verbunden werden kann, wobei die elektrische Verbindung zwischen dem Sondendraht (11a, 11b, 11c) und dem Verbinder (3) mittels eines ersten Hypotubus (13a, 13b, 13c) hergestellt wird, der an den Sondendraht (11a, 11b, 11c) gelötet ist, **dadurch gekennzeichnet, dass** der erste Hypotubus (13a, 13b, 13c) mit einem zweiten Hypotubus (15a, 15b, 15c) des elektrischen Verbinders (3) verschweißt ist.

2. Implantierbare Sonde nach Anspruch 1, wobei das erste Hypotubus (13a, 13b, 13c) teilweise in dem zweiten Hypotubus (15a, 15b, 15c) des elektrischen Verbinders (3) untergebracht ist, so dass ein Teil (L2) des ersten Hypotubus (13a, 13b, 13c) aus dem zweiten Hypotubus (15a, 15b, 15c) herausragt.

3. Implantierbare Sonde nach Anspruch 1 oder 2, wobei das erste Hypotube (13a, 13b, 13c) mit dem zweiten Hypotube (15a, 15b, 15c) des elektrischen Verbinders (3) verschweißt ist, um das erste Hypotube (13a, 13b, 13c) mit dem zweiten Hypotube (15a, 15b, 15c) elektrisch zu verbinden.

4. Implantierbare Sonde nach Anspruch 3, wobei das Löten zwischen dem mindestens einen Sondenleiter (11a, 11b, 11c) und dem ersten Hypotubus (13a, 13b, 13c) an einem ersten Ende (27) des ersten Hypotubus (13a, 13b, 13c), durch das der mindestens eine Leiterdraht (11a, 11b, 11c) eingeführt wird, und/oder an einem zweiten Ende (35) des ersten Hypotubus (13a, 13b, 13c), wobei das zweite Ende (35) dem ersten Ende (27) gegenüberliegt.

5. Implantierbare Sonde nach einem der Ansprüche 1 bis 4, umfassend eine Vielzahl von Sondenleitungen (11a, 11b, 11c), wobei jede Sondenleitung (11a, 11b, 11c) elektrisch mit einem jeweiligen ersten Hypotubus (13a, 13b, 13c) verbunden ist.

6. Implantierbare Sonde nach einem der Ansprüche 1 bis 4, die eine Vielzahl von Sondenleitungen (11a, 11b, 11c) umfasst, so dass mindestens zwei Sondenleitungen (11a, 11b, 11c) elektrisch mit derselben ersten Hypotube (13a, 13b, 13c) verbunden sind.

7. Implantierbare Sonde nach einem der vorhergehenden Ansprüche, bei der die Schweißung(en) zur Herstellung der elektrischen Verbindungen eine Laserschweißung(en) ist (sind).

8. Die implantierbare Sonde nach einem der vorhergehenden Ansprüche, wobei der mindestens eine Sondendraht ein ein- oder mehrsträngiger Sondendraht (11a, 11b, 11c) ist, so dass der Sondendraht einen Durchmesser von weniger als 150 Mikrometer oder 0,45 French hat.

9. Verfahren zum elektrischen Verbinden von mindestens einem Sondendraht (11a, 11b, 11c) einer implantierbaren Sonde (1) mit einem elektrischen Verbinder (3), wobei der elektrische Verbinder (3) so konfiguriert ist, dass er mit einer implan-tierbaren medizinischen Vorrichtung, wie einer Herzstimulations-, Defibrillations- oder/und Neuromodulationsvorrichtung, verbunden werden kann, umfassend die folgenden Schritte:
a) Der mindestens eine Fühlerdraht (11a, 11b, 11c) des Fühlers (1) ist in einem ersten Hypotubus (13a, 13b, 13c) untergebracht;
b) Den mindestens einen Fühlerdraht (11a, 11b, 11c) mit dem ersten Hypotubus (13a, 13b, 13c) elektrisch verbinden;
**gekennzeichnet durch** die folgenden Schritte:
c) das erste Hypotubus (13a, 13b, 13c) zumindest teilweise in einem entsprechenden zweiten Hypotubus (15a, 15b, 15c) des elektrischen Verbinders (3) unterzubringen;
d) Elektrisches Verbinden des ersten Hypotubus (13a, 13b, 13c) mit dem zweiten Hypotubus (15a, 15b, 15c).

10. Verfahren zum elektrischen Verbinden mindestens eines Sondendrahtes (11a, 11b, 11c) einer implantierbaren Sonde (1) mit einem elektrischen Verbinder (3) nach Anspruch 9, wobei Schritt b) und/oder d) die Durchführung einer Laserschweißung umfasst.

11. Verfahren zum elektrischen Verbinden mindestens eines Sondendrahtes (11a, 11b, 11c) einer implantierbaren Sonde (1) mit einem elektrischen Verbinder (3) nach Anspruch 9 oder 10, dessen Verlöten in Schritt b) an einem ersten Ende (27) des ersten Hypotubes (13a, 13b, 13c), durch das der mindestens eine Sondendraht (11a, 11b, 11c) eingeführt ist, und/oder an einem zweiten Ende (35) des ersten Unterrohrs (13a, 13b, 13c) erfolgt, wobei das zweite Ende (35) dem ersten Ende (27) gegenüberliegt.

12. Verfahren zum elektrischen Verbinden mindestens eines Sondendrahtes (11a, 11b, 11c) einer implantierbaren Sonde (1) mit einem elektrischen Verbinder (3) nach einem der Ansprüche 9 bis 11, wobei Schritt c) das Einführen des ersten Hypotubus (13a, 13b, 13c) in den zweiten Hypotubus (15a, 15b, 15c) umfasst, so dass ein Teil (L2) des ersten Hypotubus (13a, 13b, 13c) aus dem zweiten Hypotubus (15a, 15b, 15c) herausragt.

## Claims

1. An implantable probe comprising at least one probe wire (11a, 11b, 11c) and an electrical connector (3), the electrical connector (3) being configured to be connected to an implantable medical device such as a cardiac stimulation, defibrillation or/and neuromodulation device wherein the electrical connection between the probe wire (11a, 11b, 11c) and the connector (3) is made by means of a first hypotube (13a, 13b, 13c) soldered to the probe wire (11a, 11b, 11c) **characterised in that** the first hypotube (13a, 13b, 13c) is welded to a second hypotube (15a, 15b, 15c) of the electrical connector (3).

2. The implantable probe according to claim 1, wherein the first hypotube (13a, 13b, 13c) is partially housed in the second hypotube (15a, 15b, 15c) of the electrical connector (3) so that a portion (L2) of the first hypotube (13a, 13b, 13c) protrudes from the second hypotube (15a, 15b, 15c).

3. The implantable probe according to claims 1 or 2, wherein the first hypotube (13a, 13b, 13c) is welded to the second hypotube (15a, 15b, 15c) of the electrical connector (3) so as to electrically connect the first hypotube (13a, 13b, 13c) to the second hypotube (15a, 15b, 15c).

4. The implantable probe according to claim 3, wherein the soldering between the at least one probe wire (11a, 11b, 11c) and the first hypotube (13a, 13b, 13c) is performed at a first end (27) of the first hypotube (13a, 13b, 13c) through which the at least one conductor wire (11a, 11b, 11c) is inserted and/or at a second end (35) of the first hypotube (13a, 13b, 13c), the second end (35) being opposite the first end (27).

5. The implantable probe according to any one of claims 1 to 4, comprising a plurality of probe wires (11a, 11b, 11c), wherein each probe wire (11a, 11b, 11c) is electrically connected to a respective first hypotube (13a, 13b, 13c).

6. The implantable probe according to any one of claims 1 to 4, comprising a plurality of probe wires (11a, 11b, 11c), such that at least two probe wires (11a, 11b, 11c) are electrically connected to a same first hypotube (13a, 13b, 13c).

7. The implantable probe according to any one of the preceding claims, of which the weld(s) made to establish the electrical connections is/are a laser weld(s).

8. The implantable probe according to any one of the preceding claims, wherein the at least one probe wire is a single or multi-stranded probe wire (11a, 11b, 11c) such that the probe wire has a diameter of less than 150 micrometers or 0.45 French.

9. A method for electrically connecting at least one probe wire (11a, 11b, 11c) of an implantable probe (1) to an electrical connector (3), the electrical connector (3) being configured to be connected to an implantable medical device such as a cardiac stimulation, defibrillation or/and neuromodulation device, comprising the following steps:
a) Said at least one probe wire (11a, 11b, 11c) of the probe (1) is housed in a first hypotube (13a, 13b, 13c);
b) Electrically connect the at least one probe wire (11a, 11b, 11c) to the first hypotube (13a, 13b, 13c);
**characterised by** the following steps:
c) At least partially accommodate the first hypotube (13a, 13b, 13c) in a corresponding second hypotube (15a, 15b, 15c) of the electrical connector (3);
d) Electrically connect the first hypotube (13a, 13b, 13c) with the second hypotube (15a, 15b, 15c).

10. The method of electrically connecting at least one probe wire (11a, 11b, 11c) of an implantable probe (1) to an electrical connector (3) according to claim 9, wherein step b) and/or d) comprises performing a laser weld.

11. The method for electrically connecting at least one probe wire (11a, 11b, 11c) of an implantable probe (1) to an electrical connector (3) according to claims 9 or 10, the soldering of which in step b) is carried out at a first end (27) of the first hypotube (13a, 13b, 13c) through which the at least one probe wire (11a, 11b, 11c) is inserted and/or at a second end (35) of the first hypotube (13a, 13b, 13c), the second end (35) being opposite the first end (27).

12. The method of electrically connecting at least one probe wire (11a, 11b, 11c) of an implantable probe (1) to an electrical connector (3) according to any of claims 9 to 11, wherein step c) comprises inserting the first hypotube (13a, 13b, 13c) into the second hypotube (15a, 15b, 15c) so that a portion (L2) of the first hypotube (13a, 13b, 13c) protrudes from the second hypotube (15a, 15b, 15c).
